# EUROPÄISCHE PATENTSCHRIFT

(11) **EP 0 996 673 B1**
(45) Veröffentlichungstag und Bekanntmachung des Hinweises auf die Patenterteilung: **02.05.2003**
(21) Anmeldenummer: 98939539.7
(22) Anmeldetag: 26.06.1998
(51) Int. Cl.: C08L 3/02, C08L 3/00

(54) **THERMOPLASTISCHE MISCHUNG AUF BASIS VON STÄRKE ENTHALTEND MINDESTENS EINE KATIONISCHE UND MINDESTENS EINE ANIONISCHE STÄRKE, VERFAHREN ZU IHRER HERSTELLUNG SOWIE VERWENDUNG**
STARCH-BASED THERMOPLASTIC MIXTURE CONTAINING AT LEAST ONE CATIONIC STARCH AND AT LEAST ONE ANIONIC STARCH, METHOD FOR PRODUCING THE MIXTURE, AND USE OF THE SAME
MELANGE THERMOPLASTIQUE A BASE D'AMIDON, CONTENANT AU MOINS UN AMIDON CATIONIQUE ET AU MOINS UN AMIDON ANIONIQUE, SON PROCEDE DE FABRICATION ET SON UTILISATION

(30) Priorität: 09.07.1997 DE 19729305
(43) Veröffentlichungstag der Anmeldung: 03.05.2000
(73) Patentinhaber: Celanese Ventures GmbH, 65926 Frankfurt am Main (DE)
(72) Erfinder: BENGS, Holger, D-60598 Frankfurt (DE); WULF, Stefan, D-41066 Mönchengladbach (DE)
(74) Vertreter: Luderschmidt, Schüler & Partner
(86) Internationale Anmeldenummer: EP9803923
(87) Internationale Veröffentlichungsnummer: WO99002597

(56) Entgegenhaltungen:
- EP-A2- 0 408 501
- EP-A2- 0 408 502
- WO-A1-92/04408

## Beschreibung

Die vorliegende Erfindung betrifft eine thermoplastische Mischung auf Stärkebasis, die mindestens eine kationische und eine anionische Stärke enthält, Verfahren zu deren Herstellung sowie Verwendung der Mischung zur Herstellung von insbesondere biologisch abbaubaren Formkörpern.

Stärke als biokompatibles Material besitzt den hohen Vorteil einer grundsätzlich guten biologischen Abbaubarkeit. Zudem steht sie als sogenannter nachwachsender Rohstoff in nahezu unbegrenzter Menge zur Verfügung.

Aufgrund dieser prinzipiellen Überlegenheit gegenüber synthetischen Werkstoffen auf Rohölbasis, deren Verfügbarkeit begrenzt ist und die nur schwer und meistens unter ökologischen Nachteilen abbaubar sind, besteht ein zunehmendes Interesse, synthetische Werkstoffe durch Werkstoffe aus solchen nachwachsenden natürlichen Rohstoffen zu ersetzten.

So wurden in den letzten Jahren Verfahren entwickelt, die es ermöglichen, Stärke mittels der bekannten Kunststoffverarbeitungstechniken zu verarbeiten wie z.B. Spritzguß und Extrusion.

Verfahren zur Herstellung von thermoplastischer Stärke, die sich hervorragend zur Weiterverarbeitung mittels herkömmlicher Kunststoffverarbeitungstechniken eignet, sind z.B. in der EP 0 599 535 und WO 90/05161 beschrieben. Gemäß diesen Schriften wird vorgeschlagen, native, d.h. natürlich vorkommende, Stärke in Mischung mit sogenannter abgebauter Stärke, deren Molekülkettenlänge mittels Hydrolyse, Oxidation oder Erhitzen verkürzt worden ist, bzw. native Stärke ggf. in Mischung mit deren Derivaten, wobei keine Definition der Derivate gegeben wird, unter Zusatz von Wasser, Weichmachern und ggf. weiterer Additive und unter Einwirkung von thermischer und mechanischer Energie zu einer thermoplastischen Masse zu verarbeiten.

Die EP 0 408 502 beschreibt eine thermoplastische Mischung auf Stärkebasis, die aus chemisch nicht modifizierter Stärke zusammen mit anionisch modifizierter Stärke besteht, wobei vorgeschlagen wird, der Mischung zur Erhöhung der Formstabilität der daraus erhältlichen Formkörper wenigstens ein im wesentlichen wasserunlösliches synthetisches Polymer zuzusetzen.

Die EP 0 408 501 desgleichen Anmelders betrifft eine ähnliche Mischung, wobei jedoch die anionisch modifizierte Stärke durch eine kationisch modifizierte ausgetauscht ist.

Anwendung findet die thermoplastische Stärke zur Herstellung von Formkörpern, Folien etc., als Verpackungsmaterial, Kapseln für pharmazeutische Zwecke, Granulat, Schäume und ähnliches mehr.

Insbesondere zur Anwendung als Verpackungsmaterial auf dem Lebensmittelsektor, z.B. in Form von Schlauchfolien wie sie als Wursthüllen verwendet werden, muß das aus thermoplastischer Stärke hergestellte Produkt neben guten mechanischen Eigenschaften wie Formstabilität, Reißfestigkeit und Wasserbeständigkeit auch ausgezeichnete laterale Reißfestigkeit und gute Barriereneigenschaften gegenüber Gasen, insbesondere Luftsauerstoff und Flüssigkeiten aufweisen.

Überraschend hat sich gezeigt, daß eine thermoplastische Mischung gemäß dem Anspruch 1 die vorstehend genannten Bedingungen erfüllt.

Bevorzugte Ausführungsformen sind Gegenstand der abhängigen Produktansprüche.

Komponente A) der erfindungsgemäßen thermoplastischen Mischung auf Stärkebasis setzt sich aus wenigstens einer kationischen Stärke a) und wenigstens einer anionischen Stärke b) zusammen, wobei diese Stärken einen substitutionsgrad von 0,1 bis 3,0 aufweisen.

Komponente A) ist ein wesentlicher Bestandteil der Mischung und in dieser zu 100 Gewichtsteilen enthalten.

Unter kationischer bzw. anionischer Stärke wird üblicherweise eine chemisch modifizierte Stärke verstanden, in der Hydroxylgruppen teilweise oder auch vollständig durch anionische oder kationische Gruppen ausgetauscht sind.

Es versteht sich, daß für die Zwecke der vorliegenden Erfindung auch Stärken verwendet werden können, die entsprechende ionische Substituenten enthalten, die auf andere Weise als durch chemische Modifikation in das Polymer eingeführt worden sind.

Die für die Komponente A verwendeten Stärken können aus verschiedenen Bereichen stammen:
- rekombinante Stärken, z.B. Kartoffel- und Maisstärken mit erhöhtem Anteil an z.B. natürlich vorkommenden Phosphatgruppen,
- chemisch modifizierte Stärken, z.B. durch Oxidation von Stärke am C6-Atom oder durch Aufpropfung von ungesättigten oder gesättigten Anhydriden (Maleinsäureanhydrid, Bemsteinsäureanhydrid) oder durch nukleophile Substitution von deprotonierter Stärke mit z.B. Chloressigsäure oder durch Veresterung mit basischen Carbonsäuren (Zitronensäure, Äpfelsäure, etc.) oder durch Aufpropfung von Lactonen unter basischen Bedingungen (z.B. von ∈-Caprolacton auf Stärke).

Dabei kann insbesondere auch auf kommerziell erhältliche Stärken und Stärkederivate zurückgegriffen werden: z.B. von Avebe, Cerestar, National Starch, Purac und Südstärke.

Ein Beispiel für kommerziell erhältliche ionische Stärken sind Spezialstärke 9156 der Fa. Südstärke mit einem Borgehalt von ca. 0,6 Gew.-%. Dies entspricht in etwa einer Borsäuregruppe an jeder zehnten Glucoseeinheit.

Prinzipiell kann für die Zwecke der vorliegenden Erfindung jede anionische oder kationische Gruppe, die sich zur Modifikation von Stärke eignet, eingesetzt werden.

Beispiele für anionische Gruppen sind Carboxylgruppen, Phosphatgruppen, Sulfatgruppen. Boratgruppen, Phosphonatgruppen und Sulfonatgruppen.

Davon sind besonders bevorzugt Phosphat-, Borat- und Sulfatgruppen, von den Sulfatgruppen insbesondere diejenigen aus der Umsetzung mit Schwefelsäure.

Beispiele für kationische Gruppen sind tertiäre Aminogruppen, quartäre Ammoniumgruppen, tertiäre Phosphingruppen, quartäre Phosphoniumgruppen, Iminogruppen und Sulfoniumgruppen.

Davon sind besonders bevorzugt Amino- und Ammoniumgruppen.

Diese Gruppen können frei oder in Form ihrer Satze in dem Stärkemolekül vorliegen. Ein Stärkemolekül kann auch mit verschiedenen anionischen bzw. kationischen Gruppen substituiert sein, die zudem über verschiedene Substituenten - einführende Verbindungen und über unterschiedliche Reaktionen eingeführt sein können.

Verfahren und Verbindungen zur Einführung dieser Gruppen sind dem Fachmann geläufig und allgemein bekannt.

Im Fall von Phosphat, Sulfat oder Borat können die korrespondierenden Stärkederivate durch die Umsetzung der freien anorganischen Säuren, z.B. im Fall von Phosphat Phosphorsäure oder seinen Estern, erhalten werden.

Carboxylgruppen können z.B. über nucleophile Substitution oder einer zur Michael-Addition verwandten Variante eingeführt werden. Ein Beispiel für den ersten Reaktionstyp ist die Umsetzung von Stärke mit Chloressigsäure, ein Beispiel für den zweiten Reaktionstyp ist die Addition von Maleinsäureanhydrid an das Stärkerückgrat. Zu nennen sind u.a. auch die Umsetzung mit Hydroxycarbonsäure in einer Synthese analog zur Williamsonschen-Ethersynthese. Auf diese Weise können z.B. durch den Einsatz von Äpfelsäure, Zitronensäure oder Weinsäure mit einer Veretherungsreaktion gleichzeitig mehr als nur eine Carboxylgruppe an eine Hydroxylgruppe der Stärke angekoppelt werden.

Darüber hinaus können Verbindungen, die z.B. mindestens zwei Carboxylgruppen enthalten, wie Dicarbonsäuren etc., über Veresterung einer Carboxylgruppe mit einer Hydroxylgruppe an das Stärkerückgrat gekoppelt werden.

Kationische Stärkederivate können wie folgt erhalten werden:
Für die Ankopplung von Aminofunktionen können u.a. alle Derivate verwendet werden, die chemisch derart aktiviert sind, daß sie durch das Stärkerückgrat z.B. durch eine nucleophile Substitution, durch eine Addition oder durch eine Kondensation zur Reaktion gebracht werden. Als Beispiel für den ersten Reaktionstyp sind z.B. Trimethylammoniumchlorid oder 2-Diethylaminoethylchlorid zu nennen. Die ionische Struktur wird dabei entweder durch die direkte Umsetzung mit dem korrespondieren Salz oder durch die nachträgliche Addition von Salzsäure erhalten. Als Additionsprodukte können dabei Reaktionen mit Epoxidgruppen in der Seitengruppe des Stickstoff enthaltenden Reagenz gesehen werden. Als Beispiel sei 2,3-(Epoxypropyl)diethylammoniumchlorid oder dessen Hydrochlorid oder 2,3-(Epoxypropyl)trimethylammoniumchlorid genannt. Zur Ankopplung durch Kondensation kommt es, wenn bei der Reaktion zwischen Stärke und des die ionischen Gruppen einführenden Reagenzes zur Abspaltung von Kondensationsprodukten wie etwa Wasser oder Methanol und ähnlichem kommt.

Die Anzahl der Hydroxylgruppen pro Glucoseeinheit, die durch eine andere funktionelle Gruppe ersetzt wird, wird als Substitutionsgrad DS ("degree of substitution") bezeichnet.

Pro Glucoseeinheit sind drei freie Hydroxylgruppen vorhanden. Somit kann der Substitutionsgrad von 0,0 bis 3,0 variieren.

Beispielsweise ist bekannt, daß native Stärke per se Phosphatgruppen enthalten kann. Der Substitutionsgrad liegt hierbei im Bereich von etwa 0,001. Das bedeutet, daß rein statistisch etwa alle 300 Glucoseeinheiten eine Phosphatgruppe im Polymer vorhanden ist.

Es ist auch rekombinante Kartoffelstärke bekannt, deren Substitutionsgrad an Phosphatgruppen um den Faktor 3 gegenüber nativer Stärke erhöht ist.

Bei dem Substitutionsgrad DS handelt es sich um eine statische Größe. Ein Substitutionsgrad DS von 1,0 besagt z.B., daß im Mittel an jeder Glucoseeinheit eine Hydroxylgruppe durch einen Substituenten ersetzt ist. D.h. ein DS von 1,0 bedeutet nicht notwendigerweise, daß exakt an jeder Glucoseeinheit ein Substituent neben zwei nicht-substituierten verbleibenden Hydroxylgruppen vorliegt.

Eine kationische oder anionische Stärke im Sinne der vorliegenden Erfindung hat einen DS von 0,1 - 3,0. Vorzugsweise weist die Stärke einen DS im Bereich von 0,1 - 2,5, insbesondere von 0,1 - 1,0 und besonders bevorzugt von 0,1 bis 0,6 auf.

Der Substitutionsgrad bezieht sich hierbei auf die Gesamtsumme an kationischen bzw. anionischen Gruppen, die in der Stärke Stärkemolekül vorhanden sind.

Das Verhältnis von kationischer Stärke zu anionischer Stärke ist für die vorliegende Erfindung über den DS definiert. Im allgemeinen liegt das Mengenverhältnis in einem Bereich von DS (anionisch) = 0,2 (DS kationisch) bis DS (kationisch) = 0,2 (DS anionisch), d.h. das Verhältnis kationischer Gruppen zu anionischen Gruppen beträgt üblicherweise 0,2 - 5. Bevorzugt ist ein Verhältnis von 0,4 - 2,5. Besonders vorteilhafte Ergebnisse werden erzielt, wenn beide Stärkevarianten den gleichen bzw. einen annähernd gleichen DS aufweisen.

Je nach speziellem Verwendungszweck kann das Verhältnis jedoch auch nach oben oder unten abweichen.

Erfindungsgemäß können in der Stärke neben den anionischen bzw. kationischen Gruppen noch andere funktionelle Gruppen als Substituenten vorliegen.

Beispiele hierfür sind nichtionische Substituenten, die z.B. Ether- oder Esterfunktionen ausbilden können.

Im Fall der Anbindung weiterer Substituenten an das Stärkerückgrat über Etherverknüpfungen kommen z.B. folgende Möglichkeiten in Frage: Alkyl, wie Methyl, Ethyl, Propyl, Butyl, Alkenyl, Hydroxyalkyl, z.B. Hydroxyethyl, Hydroxypropyl. Für die Ankopplung über Estergruppen steht die Umsetzung mit Essigsäureanhydrid an oberster Stelle durch die Stärkeacetatderivate entstehen. Weitere Substituenten können durch Reaktion mit Propionsäure, Buttersäure und den höheren Fettsäuren, insbesondere aus dem natürlichen Stoffwechsel, wie etwa Laurylsäure, Ölsäure, etc., eingebracht werden.

Durch die erfindungsgemäße Kombination von anionisch modifizierter und kationisch modifizierter Stärke zur Herstellung von thermoplastischer Stärke bzw. von Formkörpern daraus, können Formkörper mit verbesserten Eigenschaften erhalten werden, wie ausreichende mechanische Festigkeit, Elastizität, Widerstandsfähigkeit gegen Wasser neben ausgezeichneter lateraler Reißfestigkeit, d.h. Reißfestigkeit senkrecht zur Verstreckrichtung der Polymerketten und hervorragenden Barriereeigenschaften gegenüber Gasen und Flüssigkeiten.

Insbesondere sind für die Verwendung als Lebensmittelverpackung gute Barriereeigenschaften unabdingbar, um z.B. Zutritt von Luftsauerstoff und damit ein Verderben der Produkte zu vermeiden.

Es wird angenommen, daß die Erhöhung der mechanischen Festigkeit eine Folge der ionischen Wechselwirkung zwischen den Polymerketten mit unterschiedlichen ionischen Gruppen ist. Es handelt sich hierbei sozusagen um eine nichtkovalente Vernetzung. Diese ist insbesondere für die thermoplastische Verarbeitung von Vorteil, da bei den erhöhten Temperaturen, bei denen die thermoplastische Verarbeitung durchgeführt wird, die ionische Wechselwirkung aufgrund der thermisch induzierten Mobilität nur geringfügig ausgebildet ist und folglich die Plastifizierung nicht behindert wird. Bei Umgebungstemperatur wird dagegen die Ausbildung, d.h. Lokalisierung bzw. Ausrichtung, der Wechselwirkungen infolge der nicht mehr vorhandenen, thermisch induzierten Mobilität, nicht mehr behindert und folglich kann der beobachtete Stabilitätseffekt bewirkt werden.

Für die kationische bzw. anionische Modifizierung können auch Stärken verwendet werden, wie sie nachstehend für die Komponente B beschrieben sind.

Das als Komponente (B) eingesetzte von Komponente (A) verschiedene thermoplastisch verarbeitbare polymere Material ist eine optionale Komponente.

Es handelt sich hierbei vorzugsweise um ein im wesentlichen biologisch abbaubares, polymeres Material, welches in Mengen bis zu 400 Gewichtsteilen bezogen auf Komponente (A) in der Mischung enthalten sein kann. Auch Mischungen von zwei oder mehrere solcher Verbindungen kommen als Komponente (B) in Frage.

Für den Fall, daß die thermoplastische Mischung zur Herstellung von Lebensmittelverpackungen etc. verwendet werden soll, wird vorzugsweise als Komponente (B) ein physiologisch verträgliches polymeres Material gewählt.

Als Komponente (B) können insbesondere auch eine oder mehrere Stärken, eine oder mehrere ihrer Derivate oder Mischungen von Stärke und Stärkederivaten verwendet werden. Es kann sich hierbei um native, chemisch modifizierte, fermentative oder rekombinante Stärke und/oder um Derivate der genannten Stärken handeln.

Eine wichtige Gruppe von Stärken umfaßt die aus pflanzlichen Rohstoffen gewonnenen Stärken. Hierzu zählen unter anderem Stärken aus Knollen, wie Kartoffeln, Maniok, Maranta, Batata, aus Samen wie Weizen, Mais, Roggen, Reis, Gerste, Hirse, Hafer, Sorghum, aus Früchten, wie Kastanien, Eicheln, Bohnen, Erbsen, u. a. Hülsenfrüchten, Bananen, sowie aus Pflanzenmark, z.B. der Sagopalme.

Die im Rahmen der Erfindung verwendbaren Stärken bestehen im wesentlichen aus Amylose und Amylopektin in wechselnden Mengenverhältnissen.

Besonders gute Ergebnisse erzielt man unter anderem mit Stärken aus Kartoffeln (z.B. Toffena der Fa. Südstärke) und Mais (z.B. Maize Starch der Fa. National Starch) oder auch Polyglucanen, die sich durch einen perfekt linearen Aufbau der Polymerketten auszeichnen.

Die Molekulargewichte der erfindungsgemäß sowohl für Komponente A) wie auch B) nützlichen Stärken können über einen weiten Bereich variieren. Einsetzbar sind solche Stärken, die im wesentlichen aus einem Gemisch von Amylose und Amylopektin bestehen, vorzugsweise mit Molekulargewichten M_{w} im Bereich zwischen 5x10⁴ und 1x10⁷. Bevorzugt werden insbesondere längerkettige Polymere mit Molekulargewichten M_{w} zwischen 1x10⁶ und 5x10⁶.

Bevorzugt werden weiterhin auch lineare Stärken, vorzugsweise Polyglucane, insbesondere 1,4-α-D-Polyglucan, mit Molekulargewichten M_{w} im Bereich zwischen 5x10² und 1x10⁵, bevorzugt zwischen 1x10³ und 5x10⁴.

Neben Stärken nativen pflanzlichen Ursprungs können auch solche Stärken, die chemisch modifiziert sind, fermentativ gewonnen wurden, rekombinanten Ursprungs sind, oder durch Biotransformation (synonym auch Biokatatyse genannt) hergestellt worden sind, verwendet werden.

Unter "chemisch modifizierten Stärken" versteht die Erfindung solche Stärken, bei denen auf chemischem Wege die Eigenschaften im Vergleich zu den natürlichen Eigenschaften verändert wurden. Dies wird im wesentlichen durch polymeranaloge Umsetzungen erreicht, bei denen Stärke mit mono-, bi- oder polyfunktionellen Reagenzien bzw. Oxidationsmitteln behandelt wird. Dabei werden vorzugsweise die Hydroxygruppen der Polyglucane der Stärke durch Veretherung, Veresterung oder selektive Oxidation umgewandelt. Eine weitere Möglichkeit besteht in einer Modifizierung, die auf einer radikalisch initiierten Propfcopolymerisation von copolymerisierbaren ungesättigten Monomeren auf das Stärkerückgrat beruht.

Zu besonderen chemisch modifizierten Stärken gehören unter anderem Stärkeester, wie Xanthogenate, Acetate, Nitrate, Stärkeether, wie z. B. nichtionische Stärkeether, oxidierte Stärken, wie etwa Dialdehydstärke, Carboxystärke, Persulfat-abgebaute Stärken und ähnliche Substanzen.

"Fermentative Stärken" sind im Sprachgebrauch der Erfindung Stärken, die durch fermentative Prozesse unter Verwendung in der Natur vorkommender Organismen, wie Pilzen, Algen oder Bakterien gewonnen werden oder unter Einschaltung und Mithilfe von fermentativen Prozessen gewonnen werden können. Beispiele für Stärken aus fermentativen Prozessen umfassen neben anderen Gum Arabicum und verwandte Polysaccharide (Gellan Gum, Gum Ghatti, Gum Karaya, Gum Tragacauth), Xanthan, Emulsan, Rhamsan, Wellan, Schizophyllan, Polygalacturonate, Laminarin, Amylose, Amylopektin und Pektine.

"Stärken rekombinanten Ursprungs" oder "rekombinante Stärken" meint im einzelnen Stärken, die durch fermentative Prozesse unter Verwendung in der Natur nicht vorkommender Organismen, aber unter Zuhilfenahme von gentechnischen Methoden modifizierten natürlichen Organismen, wie Pilzen, Algen oder Bakterien gewonnen werden oder unter Einschaltung und Mithilfe von fermentativen Prozessen gewonnen werden können. Beispiele für Stärken aus fermentativen, gentechnisch modifizierten Prozessen sind neben anderen Amylose, Amylopektin und weitere Polyglucane.

"Durch Biotransformation hergestellte Stärken" bedeutet im Rahmen der Erfindung, daß Stärken, Amylose, Amylopektin oder Polyglucane durch katalytische Reaktion von monomeren Grundbausteinen, im allgemeinen oligomeren Sacchariden, insbesondere Mono- und Disacchariden, hergestellt werden, indem ein Biokatalysator (auch: Enzym) unter speziellen Bedingungen verwendet wird. Beispiele für Stärken aus biokatalytischen Prozessen sind neben anderen Polyglucan und modifizierte Polyglucane, Polyfructan und modifizierte Polyfructane.

Schließlich lassen sich auch unter Verwendung von Derivaten der einzelnen genannten Stärken vorteilhafte thermoplastische Mischungen erhalten. Dabei bedeuten die Begriff "Derivate von Stärken" oder "Stärkederivate" ganz allgemein modifizierte Stärken, d. h. solche Stärken, bei denen zur Veränderung ihrer Eigenschaften das natürliche Amylose/Amylopektin-Verhältnis verändert wurde, eine Vorverkleisterung durchgeführt wurde, die einem partiellen hydrolytischen Abbau unterzogen wurden oder die chemisch derivatisiert wurden.

Besonders günstige thermoplastische Mischungen werden auch erhalten, wenn als Komponente (B) Stärken eingesetzt werden, die als solche einen möglichst geringen Anteil anderer Verbindungen, die nicht den Sacchariden zuzurechnen sind (z.B. Proteine, Fette, Öle), aufweisen wie z.B. Kartoffelstärke und/oder durch ihre Einheitlichkeit in bezug auf Struktur, Molekulargewicht und Reinheit herausragende biokatalytisch erzeugte Polyglucane verwendet werden.

Zu im Rahmen der Erfindung mit Erfolg einsetzbaren Komponenten (B) gehören aber auch Proteine. Beispiele dafür sind unter anderem Gelatine, pflanzliche Proteine, wie Sonnenblumenprotein, Sojaprotein, Weizenprotein, Baumwollsamenprotein, Erbsenprotein, Erdnußprotein, Rapssamenprotein, Plasmaproteine, Eiweiß, Eigelb und ähnliche.

Günstige Mischungen ergeben auch Zusätze von Zein, Gluten (Mais, Kartoffel), Albumin, Casein, Kreatin, Kollagen, Elastin, Fibroin und/oder Molkeprotein.

Von Interesse als Komponente B) sind zudem Polysaccharide.
Vorzugsweise werden wasserlösliche Polysaccharide wie Alginsäure und ihre Salze, Carrageenane, Furcellaran, Guar Gum, Agar-Agar, Gum Arabicum und verwandte Polysaccharide (Gum Ghatti, Gum Karaya, Gum Tragacauth), Tamarind Gum, Xanthan Gum, Aralia Gum, Johannesbrot Gum ('locust bean gum'), Arabinogalactan, Pullulan, Chitosan, Dextrine, Cellulose eingesetzt.

Günstig können sich auch Zusätze von Lentinan, Laminarin, Chitin, Heparin, Inulin, Agarose, Galactane, Hyaloronsäure, Dextranen, Dextrinen, Poly-∈-caprolacton und/oder Glykogen auswirken.

Die erfindungsgemäße thermoplastische Mischung wird rechnerisch bezüglich der Komponenten (A) und (B) auf einen Wassergehalt von Null Prozent korrigiert. D. h., der Wassergehalt der Komponenten (A) und (B) wird bestimmt und bei der Bemessung der eingesetzten Gewichtsteile entsprechend abgezogen, aber bei der Bemessung der Komponente © berücksichtigt.

Die Komponente (C), Wasser, der erfindungsgemäßen Mischung ist eine essentielle Komponente.

Die Menge an Wasser, die für die Plastifizierung notwendig ist, kann je nach Art der eingesetzten Mischung in einem weiten Bereich variieren.

Ist die Menge an zugesetztem Wasser zu gering, so ist die Destrukturierung und Homogenisierung der Mischung unzureichend. Ist der Wassergehalt zu hoch, besteht die Gefahr, daß die Viskosität der Mischung zu niedrig ist.

Im allgemeinen ist Wasser in einer Menge von 1 Gew.-Teil bis zu Dreiviertel, insbesondere bis zur Hälfte, der Summe der Gewichtsteile (A) und (B) in der Mischung der Erfindung ausreichend.
Bevorzugte Wassergehalte liegen etwa zwischen 5 und ((A)+(B))/1,3 Gewichtsteilen, besonders bevorzugt sind Wasseranteile zwischen 10 und ((A)+(B))/1,3 Gewichtsteilen.

In diesen bevorzugten Bereichen findet eine optimale Plastifizierung der Mischung, d. h. Destrukturierung der Stärke, Homogenisierung der Mischung sowie deren Thermoplastifizierung statt.

Die Wassermenge (C) beinhaltet neben tatsächlich zugefügtem Wasser auch die rechnerisch zu berücksichtigenden Wassergehalte anderer Komponenten, insbesondere die Menge von in den Komponenten (A) und (B) gebundenem oder enthaltenem Wasser.

Die Natur der Komponente (C) ist im wesentlichen nicht weiter kritisch. Man kann VE-Wasser, deionisiertes Wasser aber auch genauso gut Leitungswasser oder Wasser anderen Ursprungs einsetzen, sofem der Gehalt des Wassers an Salzen oder anderen Fremdstoffen im Hinblick auf die beabsichtigte Verwendung tolerabel ist.

Die Komponente (D) ist essentiell in der erfindungsgemäßen Mischung enthalten.

Ein oder mehrere Weichmacher sind in der Komposition der Erfindung in einer Menge im Bereich von 10 Gew.-Teilen bis zur Hälfte der Summe der Gewichtsteile (A) und (B) enthalten. Liegt der Gehalt an weichmachenden Verbindungen unterhalb von 10 Gewichtsteilen, so ist die Ptastifizierung nicht ausreichend, selbst bei höheren mechanischen und/oder thermischen Energien. Überschreitet der Weichmachergehalt eine Menge die der Hälfte der Summe der Gewichtsteile (A) und (B) entspricht, wird keine nennenswert bessere Plastifizierung der Mischung beobachtet.

Günstig sind Weichmachermengen im Bereich von 12,5 bis ((A)+(B))/2 Gewichtsteilen, besonders bevorzugt sind Gehalte an Weichmachern im Bereich von 15 bis ((A)+(B))/4 Gewichtsteilen.

Der jeweils optimale Gehalt an Weichmacher richtet sich nach den übrigen Komponenten und sollte zweckmäßiger Weise für jede Formulierung separat bestimmt werden.

Grundsätzlich sind im Rahmen der Erfindung die Begriffe Weichmachungs-, Plastifikations-, Plastifizierungs-, oder Elastifizierungsmittel gleichbedeutend mit Weichmacher.

Einsetzbar sind alle indifferenten, vorzugsweise organischen, Substanzen mit im allgemeinen geringem Dampfdruck, welche ohne chemische Reaktion, vorzugsweise durch ihr Löse- und Quellvermögen den Komponenten (A) und gegebenenfalls (B) in physikalische Wechselwirkung treten und ein homogenes System mit diesen bilden.

Die erfindungsgemäß einzusetzende Komponente (D) verleiht der Mischung vorzugsweise eine erniedrigte Einfriertemperatur, erhöhtes Formveränderungsvermögen, erhöhte elastische Eigenschaften, verringerte Härte und ggf. gesteigertes Haftvermögen.

Bevorzugte Weichmacher gemäß der Erfindung sind geruchlos, farblos, licht-, kälteund wärmebeständig, nur wenig bis gar nicht hygroskopisch, wasserbeständig, nicht gesundheitsschädlich, schwer brennbar und möglichst wenig flüchtig, neutral reagierend, mit Polymeren und Hilfsstoffen mischbar und weisen ein gutes Gelierverhalten auf. Insbesondere sollen sie gegenüber den Komponenten A) und gegebenenfalls B) Verträglichkeit, Geliervermögen und weichmachende Wirksamkeit aufweisen.

Weiter sollen die erfindungsgemäß als Komponente D) einzusetzenden Verbindungen eine geringe Migration aufweisen, was insbesondere für Anwendungen der erfindungsgemäßen Formkörper im Lebensmittelbereich von Bedeutung ist.

Zu besonders bevorzugten weichmachenden Komponenten D) gehören unter anderem Dimethylsulfoxid, 1,3-Butandiol, Glyzerin, Ethylenglykol, Propylenglykol, Diglyzerid, Diglycolether, Formamid, N,N-Dimethylformamid, N-Methylformamid, Dimethylacetamid, N-Methylacetamid und/oder N,N'-Dimethylharnstoff.

Besonders vorteilhaft sind auch Polyalkylenoxide, Glycerinmono-, di-, oder -triacetat, Sorbitol, oder andere Zuckeralkohole, wie Erythrit, Zuckersäuren, Saccharide, wie Glucose, Fructose oder Saccharose, sowie Zitronensäure und seine Derivate.

Die Komponente (E) der erfindungsgemäßen Mischung ist optional. Es kann sich um einen oder mehrere Stoffe handeln, welche insgesamt als Komponente (E) in Mengen bis zu ((A)+(B)) Gewichtsteilen, vorzugsweise nicht mehr als ((A)+(B))/2 Gewichtsteilen, einsetzbar sind.

Zu üblichen Zuschlagstoffen oder Additiven gehören unter anderem Füllstoffe, Gleitmittel, die von den unter (D) genannten Weichmachern verschieden sind, Flexibilisierungsmittel, Pigmentierungsmittel, Farbstoffe, Entformungsmittel und andere.

Als Füllstoff geeignet sind beispielsweise synthetische Polymere, die nahezu in der Mischung löslich sind, wie etwa auf Milchsäure basierende Polymere, wie ®Lacea der Firma Mitsui, ®Resomer der Firma Boehringer Ingelheim, sowie weitere Polymere auf der Basis von Milchsäure und artverwandte Polymere der Milchsäure, der Firmen Wako Pure Chemical Industries Ltd., Medisorb Co., Birmingham Polymers, Inc., Polysciences inc., Purac Biochem BV, Ethicon, Cargill oder Chronopol, wobei einsichtig ist, daß diese Aufzählung nicht einer absoluten Vollständigkeit entsprechen kann oder Blends von synthetischen Polymeren mit natürlichen Polymeren z.B. Mater-Bi der Fa. Novamont.

Bei Bedarf können auch ein oder mehrere anorganische Füllstoffe, wie beispielsweise Magnesiumoxid, Aluminiumoxid, SiO₂, TiO₂ usw. zugesetzt werden.

Zum Einfärben der Mischung eignen sich insbesondere organische oder anorganische Pigmente, besonders auch Perlglanzpigmente, die überwiegend auf Silikatstrukturen basieren, und daher biokompatibel sind, also als unbedenklich für lebende Organismen und prinzipiell als eßbar einzustufen sind und in Mengen zwischen 0,001 und 10 Gewichtsteilen eingesetzt werden können.

Zur Verbesserung der Fliesseigenschaften eignen sich insbesondere tierische oder pflanzliche Fette und/oder Lecithine, die vorzugsweise in hydrogenierter Form verwendet werden, wobei diese Fette und sonstigen Fettsäurederivate vorzugsweise einen Schmelzpunkt von größer 50 °C aufweisen.

Um die Wasserbeständigkeit der thermoplastisch verarbeitbaren Mischung während und nach der Verarbeitung zu erhöhen, kann dem Gemisch ein Vemetzungsmittel in untergeordneten Mengen zugesetzt werden, um die Stärke chemisch zu modifizieren. Vorzugsweise werden hierzu Alkylsitoxane in Mengen bis zu 5 Gewichtsteilen eingesetzt.

Als Vernetzungsmittel eignen sich unter anderem auch zwei- oder mehrwertige Carbonsäuren sowie deren Anhydride, Säurehalogenide von zwei- oder mehrwertigen Carbonsäuren, Säureamide von zwei- oder mehrwertigen Carbonsäuren, Derivate von zwei- oder mehrwertigen anorganischen Säuren, Dialdehyde, insbesondere Glyoxal und Glutardialdehyd, Epoxide, Diepoxide, Ethylenglycoldiglycidylether, Formaldehyd und/oder Harnstoffderivate, Divinylsulfone, Diisocyanate, Isocyanate, Oxoverbindungen und/ Cyanamid, wobei sich diese Verbindungen auch besonders zur chemischen Modifizierung im Anschluß an die thermoplastische Verarbeitung eignen und somit zur weiteren Verbesserung insbesondere der mechanischen Eigenschaften beitragen können.

Die für die einzelnen Komponenten (E) angegebenen Gewichtsteile können je nach Bedarf variieren.

Die Komponenten (A) bis (E) der erfindungsgemäßen Mischung werden ggf. unter Einbringung von thermischer und/oder mechanischer Energie vermischt und unter Einbringung von thermischer und/oder mechanischer zu einer thermoplastischen Mischung verarbeitet.

Vorzugsweise erfolgt die Einbringung der mechanischen und der thermischen Energie gleichzeitig, z. B. durch Arbeiten unter erhöhter Temperatur und gleichzeitiger Ausübung von Scherkräften auf die zu plastifizierende thermoplastische Mischung auf Stärkebasis.

Im allgemeinen gilt, daß eine bessere Homogenität der Mischungen bei höheren Temperaturen resultiert. Allerdings sollen die Temperaturen nicht zu hoch liegen, um unnötige Verfärbungen oder Zersetzung der Formmassen zu vermeiden.

In diesem Zusammenhang ist die thermoplastische Mischung der Erfindung in bevorzugter Abwandlung erhältlich durch Mischen bei Temperaturen im Bereich von > 60 °C bis 220 °C, vorzugsweise 80°C bis 180°C und besonders bevorzugt 100 °C bis 160°C.

Grundsätzlich steigt die Homogenisierung der Mischung mit der eingebrachten Leistung an. D. h. je höher die eingebrachte Leistung in das Mischaggregat ist, um so besser erfolgt die Homogenisierung der thermoplastischen Stärkemischung.

Es ist jedoch darauf zu achten, daß die über das Mischaggregat eingebrachte mechanische Energie nicht in einem zu großen Maße in thermische Energie umgesetzt wird, was zu einer erwünschten Temperaturerhöhung führen kann. Zur Vermeidung können geeignete Kühlthermostate eingesetzt werden.

Eine weitere Modifikation der Erfindung sieht eine durch Mischen unter Einwirkung von stark scherenden Mischaggregaten erhältliche thermoplastische Mischung vor, wobei die in die Mischung eingebrachte Energie insbesondere an der Leistung der verwendeten Verarbeitungsmaschinen abgeleitet werden kann. So ist eine Verarbeitung vor allem mit Apparaturen möglich, deren Plastifizierungselement mit Drehmomenten ausgestattet sind, die im Bereich von 5 bis 300 Nm (1 Newton Meter) liegen. Als vorteilhaft hat sich eine Verarbeitung bei einem Drehmoment im Bereich von 10 bis 100 Nm erwiesen. Bevorzugt wird die Verarbeitung in einem Bereich des Drehmomentes von 20 bis 40 Nm.

Eine besonders günstige Aufnahme von thermischer und/oder mechanischer Energie durch die Mischung wird erreicht, wenn man die Bestandteile der Mischung gemäß der Erfindung in einer Kunststoffverarbeitungsmaschine, wie beispielsweise einem Extruder, Kneter oder ähnlichen Aggregaten mischt und homogenisiert.

Das Verfahren kann vorzugsweise auf Ein-oder Zweischneckenextrudern durchgeführt werden. Diese sind vorzugsweise aus einzelnen Gehäusen zusammengesetzt, die temperierbare Mäntel aufweisen. Die Gestaltung der Schnecken unterliegt keiner Beschränkung, es können Förderelemente mit oder ohne Schubkanten, Knetelemente und/oder Mischelemente vorhanden sein. Darüber hinaus ist es möglich und häufig vorteilhaft zumindest teilweise, d. h. abschnittsweise stauende oder rückfördernde Elemente im Extruder zu verwenden, um Verweilzeit und Mischungseigenschaften zu beeinflussen und zu steuern.

Im allgemeinen hat die Reihenfolge der Zumischung der Komponenten (A) bis (F) keinen besonderen Einfluß auf die Eigenschaften der erhaltenen thermoplastischen Mischung.

Die erfindungsgemäße thermoplastische Formmasse läßt sich nach den bekannten Verarbeitungsverfahren zu Produkten verarbeiten. So kann sie z. B. in einem ersten Schritt granuliert oder pelletisiert werden.

Gegenstand der Erfindung ist somit auch ein Granulat, das durch Extrusion und Pelletisierung aus der thermoplastischen Mischung gemäß der Erfindung erhältlich ist.

Außerdem können entweder direkt oder durch erneutes thermoplastisches Verarbeiten des sich thermoplastisch verhaltenden Granulats biologisch gut abbaubare Formteile oder Folien mit verbesserten mechanischen Eigenschaften erhalten werden.

Schließlich gehört zur Erfindung insbesondere auch die Verwendung der thermoplastischen Mischungen zur Herstellung von Formteilen oder Folien.

Insgesamt decken die erfindungsgemäßen Produkte damit eine Vielzahl von Anwendungsmöglichkeiten ab. Hierzu gehören im einzelnen unter anderem Klebstoffadhäsive für Papier und Wellpappe, Formkörper, die durch Spritzguß hergestellt werden, vor allem Stäbe, Rohre, Flaschen, Kapseln, Granulate, Lebensmittelzusatzstoffe, Filme, als Überzüge oder freistehende Filme, auch als Laminate, vor allem Folien, Verpackungsmaterialien, Beutel, Retardmaterialien zur kontrollierten Freisetzung von Wirkstoffen im allgemeinen, insbesondere Pharmaka, Pestizide oder andere in der Agrokultur eingesetzte Wirkstoffe, Dünger, Aromastoffe etc. Dabei kann die Freigabe der aktiven Substanz aus Filmen, Folien, Preßlingen, Partikeln, Mikropartikeln, Stäbchen oder anderen Extrudaten oder sonstigen Formkörpern erfolgen.

Die aus der erfindungsgemäßen thermoplastischen Mischung erhaltenen Produkte, wie Formkörper oder Folien, sind im wesentlichen biokompatibel und bei Verwendung von entsprechend ausgewählten Komponenten eßbar, was den Weg zu eßbaren Verpackungen, also insbesondere Lebensmittelverpackungen, ebnet.

Unter Lebensmittelverpackungen sind dabei sowohl Umverpackungen zu verstehen, die mit dem Lebensmittel nur temporären Kontakt haben, als auch Verpackungen, wie Schläuche, Hüllen oder Überzüge, die an ihrer Innenoberfläche ständigen Kontakt mit dem Lebensmittel haben und daher auch bei der Nahrungsmittelaufnahme aufgenommen werden können. Die Verpackungen eignen sich daher neben anderen für Obst, Eier, Käse, Bonbonwaren, Kuchen, Kekse oder Brausetabletten, Getränke, Fleisch, Wurstwaren und Fleischbrät.

Der Einsatz der aus den thermoplastischen Formmassen gemäß der Erfindung erhältlichen Formkörper ist dabei nicht auf die Verwendung in Kombination mit temporären Produkten beschränkt, sondern kann auch auf den temporären Einsatz zum Schutz von Gebrauchsgegenständen und Investitionsgütern bei Transport oder Lagerung angewendet werden. Insbesondere ist hier an den Schutz vor klimatischen Einflüssen zu denken, wie sie etwa beim überseeischen Transport von Automobilen auftreten.

Weiter bevorzugte Anwendungen umfassen Absorber, Puder und dergleichen.

In einer besonderen Ausführungsform werden die thermoplastischen Mischungen gemäß der Erfindung zur Herstellung von Formkörpern zur kontrollierten Freigabe von Wirkstoffen, wie etwa Tabletten oder Dragees, verwendet.

Eine weitere zweckmäßige und besonders günstige Verwendung der thermoplastischen Mischung gemäß der Erfindung betrifft die Herstellung von Formkörpern, die sich zur Herstellung von massiven Formkörpern, Hohlkörpern oder Kombinationen davon eignen.

Noch eine herausragende Verwendung der erfindungsgemäßen thermoplastischen Mischung ist in der Herstellung von Folien zum Gebrauch in der Landwirtschaft angesiedelt.

In weiterer besonderer Abwandlung sieht die Erfindung die Verwendung der thermoplastischen Mischung zur Herstellung von Folien zum Gebrauch für Lebensmittelanwendungen vor.

Eine spezielle erfindungsgemäße Verwendung der thermoplastischen Mischung liegt in der Herstellung von Folien zum Gebrauch als Lebensmittelumverpackung.

Eine weiterhin bevorzugte günstige Verwendung der thermoplastischen Mischung gemäß der Erfindung ergibt sich bei der Herstellung von Folien zum Gebrauch als Lebensmittelverpackung mit vollständigem Flächenkontakt zum Lebensmittel.

Schließlich ist auch eine Verwendung der thermoplastischen Mischung gemäß der Erfindung besonders vorteilhaft bei der Flach- oder tubulare Folien zur Verwendung als Lebensmittelhüllen für Wurst und Käse hergestellt werden.

Zur besonderen Anpassung an den jeweiligen Verwendungszweck können der erfindungsgemäßen thermoplastischen Mischung oder den daraus erhaltenen Granulaten bei der Herstellung der Formkörper nach Bedarf weitere geeignete Materialien zugesetzt werden.

Derartige Materialien sind an sich bekannt. Beispiele dafür sind Fasern, Vernetzungsmittel, Proteine, Hydrophobisierungsmittel, Gleitmittel, synthetische Kunststoffe etc.

Die Mengenangaben beziehen sich auf das Gewicht der eingesetzten Stärke, d.h. Gewicht der Komponente (A) und ggf. (B). Je nach Bedarf können diese Angaben jedoch variieren.

Beispielsweise können zur Erhöhung der mechanischen Festigkeit Fasern in einer Menge von 5-70 Gew.-%, vorzugsweise 20-45 Gew.-%, wie Baumwollfasern, Hanffasern, Cellulose etc. beigemengt werden.

Als Vernetzungsmittel können dieselben wie vorstehend in Zusammenhang mit der Plastifizierung beschrieben verwendet werden. Bevorzugt sind z.B. Dicarbonsäuren, Dialdehyde, insbesondere Glyoxal und Glutardialdehyd, Diisocyanate und Diepoxide, z.B. Ethylenglykoldiglydiylether oder auch Polyphosphate.

Die Vernetzungsmittel tragen zur Verbesserung der Wasserbeständigkeit bei. Im allgemeinen werden sie in einer Menge von 0,1 bis 10 Gew.-%, vorzugsweise 0,5 - 3 Gew.-% verwendet.

Auch Proteine wie sie z.B. vorstehend beschrieben worden sind können zugesetzt werden, insbesondere Casein, Gelatine, Soja-, Weizen und Erbsenprotein. Die zugesetzte Menge ist im allgemeinen 2 - 40 Gew.-%, vorzugsweise 3 - 10 Gew.-%.

Als weitere Zusatzstoffe sind übliche Hydrophobisierungsmittel und/oder Gleitmittel zu nennen, die im allgemeinen in einer Menge von 2 - 12 Gew.-% vorzugsweise 3 - 6 Gew.-% eingesetzt werden.

Durch den Zusatz von beispielsweise Gleitmitteln kann die Schälbarkeit von Lebensmittelverpackungen, z.B. Wursthüllen, verbessert werden. Auch üben sie einen positiven Effekt auf die Wasserbeständigkeit aus.

Weichmacher, wie die vorstehend beschrieben, z.B. Glycerin oder Citronensäure, können in üblichen Mengen, z.B. 5 - 40 Gew.-%, vorzugsweise 5 - 20 Gew.-% zugesetzt werden.

Durch den Zusatz von Weichmachern läßt sich beispielsweise die Geschmeidigkeit von Verpackungen, insbesondere z.B. für Wursthüllen, erhöhen.

Als weitere mögliche Zusatzstoffe kommen synthetische Polymere in Frage. Geeignete Beispiele sind weiche und zähe Polyamide, Polyester, Polyolefine, Ethylen/Acrysäureester/Maleinsäureanhydridcopolymere oder Polyvinylpyrrolidon.

Bevorzugte Polyolefine sind Hochdruckpolyethylen oder Polypropylen. Der Anteil an synthetischen Polymeren beträgt zweckmäßigerweise 5 - 50 Gew.-%, vorzugsweise 10 - 40 Gew.-%.

Die aus der erfindungsgemäßen thermoplastischen Mischung erhaltenen Formkörper können mit für aus Biopolymeren hergestellten Formkörpern oder Folien bekannten Maßnahmen bearbeitet oder kombiniert werden. Beispielsweise sind die für Cellulosehydrathüllen bekannten Imprägnierungen oder Beschichtungen auf die gemäß der Erfindung erhaltenen Formkörper bzw. Folien anwendbar. Dies betrifft insbesondere auch die Verwendung als Lebensmittelhüllen.

Die folgenden Beispiele dienen lediglich der Veranschaulichung der Erfindung.

### Beispiel 1

Herstellung eines thermoplastisch verarbeitbaren Blendgemisches aus ionischen Stärken:
Die Mischungen werden in einem Knetaggregat (Brabender Kneter) hergestellt. Das Knetaggregat wird auf Temperaturen zwischen 100 °C und 140 °C aufgeheizt. Die Heiztemperaturen sind dabei abhängig von der eingesetzten Mischung der verschiedenen Stärken sowie der weiteren zugesetzten Additive auf der Basis von Polymeren, Weichmachern, Modifizierungsmittel, Vernetzer etc.
Ein typisches Knetexperiment ist im folgenden beschrieben. 15 g kationische Kartoffelstärke (Fibraffin K5 der Fa. Südstärke) und 15 g anionische Kartoffelstärke (Fibraffin A5 der Fa. Südstärke) werden während des Betriebszustands in das Knetaggregat gegeben. 15 g Wasser werden im Anschluß in dünnem Strahl hinzugegeben. Die Öffnung des Kneters wird mit einer Metallplatte abgedeckt, um ein zu frühes Verdampfen des Wassers zu verhindern. Es werden nach ca. 10 Minuten 9 g Glyzerin hinzugegeben. Nach einer weiteren Homogenisierungsphase kann das Produkt entweder im noch erwärmten Zustand dem Gerät entnommen und weiter verarbeitet werden.

### Beispiel 2

Herstellung eines thermoplastisch verarbeitbaren Blendgemisches aus ionischen Stärken:
Die Durchführung erfolgt wie in Beispiel 1 beschrieben, jedoch mit dem Unterschied, daß nach der Wasserzugabe und der sich anschließenden Homogenisierungsphase 3 g Glyoxal hinzugegeben werden. Nach einer weiteren Homogenisierungsphase kann das Produkt entweder im noch erwärmten Zustand des Gerätes entnommen und weiter verarbeitet werden.

### Beispiel 3

Kommerzielle Beispiele ionischer Stärken für die Herstellung von thermoplastischen Materialien:
Neben synthetischen ionischen Stärken können kommerziell erhältliche Stärken verschiedener Hersteller eingesetzt werden. Die nachfolgende Tabelle gibt eine Auflistung der vorwiegend eingesetzten Stärken wieder. Jedoch können vergleichbare Ergebnisse mit ionischen Stärken anderer Hersteller erzielt werden.

| Bezeichnung | Hersteller | Ausgangsprodukt | Löslichkeit | lonenart | Struktur |
|---|---|---|---|---|---|
| Cato 230 | National Starch | Mais | heißwasserlöslich | kationisch | Amin-modifikation von Wachsmaisstärke |
| Catsol 12 SSG | Südstärke | Kartoffel | kaltwasserlöslich | kationisch | Amonium |
| Catsol 9566 - SSG K8 | Südstärke | Kartoffel | kaltwasserlöslich | kationisch | Amonium |
| Fibraffin K102 | Südstärke | Kartoffel | heißwasserlöslich | kationisch | Amonium |
| Fibraffin K3 | Südstärke | Kartoffel | heißwasserlöslich | kationisch | Amonium |
| Fibraffin K5 | Südstärke | Kartoffel | heißwasserlöslich | kationisch | Amonium |
| Fibraffin K7 | Südstärke | Kartoffel | heißwasserlöslich | kationisch | Amonium |
| Licocat P | Südstärke | Kartoffel | Suspension | kationisch | quatäre kationische muitivalente Seitengruppen (hochkationisch) |
| Licosoft | Südstärke | Kartoffel | Suspension | kationisch | quatäre kationische multivalente Seitengruppen + hydrophoben Alkylgruppen (mittelkationisch) |
| Dispergiermittel | Südstärke | Kartoffel | 33%ige Lösung | anionisch | hochanionsch, Ladungen: Carboxyl-, Sulfonsäure-, und Acrylsäuregruppen |
| Fibraffin A5 | Südstärke | Kartoffel | heißwasserlöslich | anionisch | quartärer Stärkeether |
| Spezialstärke 9156 | Südstärke | Kartoffel | kaltwasserlöslich | anionisch | boriert |
| Cato 245 | National Starch | Mais | heißwasserlöslich | biionisch | Wachsmaisstärke mit Ester- und etherseitengruppen |
| Fibraffin KA 504 | Südstärke | Kartoffel | heißwasserlöslich | biionisch | Amonium |

### Beispiel 4

| Versuchs-Nr. | Stärke (100 Teile) | Polymer-zusatz (%) | Wasser (%) | Glyzerin (%) | Vemetzer (%) | Reißfestigkeit (N/mm²) | Reißdehnung (%) |
|---|---|---|---|---|---|---|---|
| 1 | Fibraffin A5 | (a) 0 | 50 | 30 | Glyoxal 10 | 21,0 | 4,0 |
| 2 | Fibraffin K5 | (b) 0 | 50 | 30 | Glyoxal 10 | 36,5 | 8,0 |
| 3 | Fibraffin A5 | Fibraffin K5 | 50 | 30 | Glyoxal 10 | 39,5 | 6,0 |
| 4 | Fibraffin A5 | Fibraffin K5 50 | 50 | 30 | Glyoxal 10 | 21,7 | 24,9 |
| 5 | Fibraffin K5 | Fibraffin A5 20 | 50 | 30 | Glyoxal 10 | 14,8 | 66,0 |
| 6 | Fibraffin K5 | Spezialstärke 9156 (a) 50 | 50 | 30 | Glyoxal 10 | 26,0 | 22,1 |
| 7 | Fibraffin A5 | Fibraffin K5 50 | 50 | 30 | Glyoxal 1 | 8,6 | 110,0 |
| 8 | Fibraffin A5 | Fibraffin K5 50 | 50 | 30 | Glyoxal 0,5 | 6,0 | 150,0 |
| 9 | Fibraffin A5 | Fibraffin K5 50 | 50 | 30 | Glyoxal 0,1 | 9,3 | 71,0 |
| 10 | Fibraffin A5 | Fibraffin K5 50 Dialdehydstärke 10 | 50 | 30 | Glyoxal 10 | 9,6 | 111,0 |

| | | | | | | | |
|---|---|---|---|---|---|---|---|
| (a) anionisch | | | | | | | |
| (b) kationisch | | | | | | | |

## Patentansprüche

1. Thermoplastische Mischung auf Stärkebasis erhältlich durch Bereitstellen und Mischen von
(A) 100 Gewichtsteile wenigstens einer kationischen Stärke a) und wenigstens einer anionischen Stärke b), wobei diese Stärken einen Substitutionsgrad DS von 0,1 - 3,0 aufweisen.
(B) 0 - 400 Gewichtsteilen eines thermoplastisch verarbeitbaren von A) verschiedenen polymeren Materials,
(C) Wasser in einer Menge, die für die Plastifizierung der Mischung ausreichend ist
(D) mindestens einen Weichmacher in einer Menge von 10 Gewichtsteilen bis zur Hälfte der Summe der Gewichtsteile A) und B), und
(E) gegebenenfalls bis zu ((A) + (B)) Gewichtsteilen weiterer üblicher Zusätze,
wobei der Wassergehalt der Komponenten A) und B) rechnerisch auf Null korrigiert wurde.

2. Thermoplastische Mischung nach Anspruch 1, wobei die anionischen Gruppen ausgewählt sind unter Carboxyl-, Phosphat-, Sulfat-, Borat-, Phosphonium- und Sulfonatgruppen.

3. Thermoplastische Mischung nach Anspruch 1 oder 2, wobei die kationischen Gruppen ausgewählt sind unter tertiären Amino-, quartären Ammonium-, tertiären Phosphin-, quartären Phosphonium-, Imino- und Sulfoniumgruppen.

4. Thermoplastische Mischung nach einem der Ansprüche 1 bis 3, wobei das Verhältnis von kationischen Gruppen zu anionischen Gruppen 0,2 - 5 ist.

5. Thermoplastische Mischung nach einem der Ansprüche 1 bis 4, wobei die Stärke zusätzlich zu den anionischen bzw. kationischen Gruppen, nicht-ionische Substituenten enthält.

6. Verfahren zur Herstellung einer thermoplastischen Mischung auf Stärkebasis, bei dem man
(A) 100 Gewichtsteile wenigstens einer kationischen Stärke a) und wenigstens einer anionischen Stärke b), wobei diese Stärken einen Substitutionsgrad DS von 0,1 - 3,0 aufweisen.
(B) bis zu 400 Gewichtsteile eines thermoplastisch verarbeitbaren von (A) verschiedenen polymeren Materials,
(C) Wasser in einer Menge, die für die Plastifizierung der Mischung ausreichend ist,
(D) mindestens einen Weichmacher in einer Menge von 10 Gewichtsteilen bis zur Hälfte der Summe der Gewichtsteile (A) und (B), und
(E) gegebenfalls bis zu ((A)+(B)) Gewichtsteilen weiterer üblicher Zusätze, wobei der Wassergehalt der Komponenten (A) und (B) rechnerisch auf Null korrigiert wurde,
bereitstellt und miteinander vermischt und die Plastifizierung unter Einbringung von thermischer und mechanischer Energie, vorzugsweise erhöhter Temperatur und gleichzeitiger Ausübung von Scherkräften, erfolgt.

7. Verfahren nach Anspruch 6, wobei die Plastifizierung bei Temperaturen im Bereich von > 60°C bis 200°C erfolgt.

8. Verfahren nach Anspruch 6 oder 7, wobei das Mischen und/oder Plastifizieren unter Einwirkung von stark scherenden, Plastifizierungselemente aulweisenden Mischaggregaten erfolgt, und die Plastifizierungselemente Drehmomente im Bereich von 10 bis 100 Nm aufweisen.

9. Granulat erhältlich aus der thermoplastischen Mischung gemäß den Ansprüchen 1 bis 5 durch Extrusion und Pelletisierung.

10. Biologisch abbaubares Formteil oder Folie erhältlich aus der thermoplastischen Mischung gemäß den Ansprüchen 1 bis 5.

11. Verwendung der thermoplastischen Mischung gemäß den Ansprüchen 1 bis 5 zur Herstellung von Formteilen oder Folien.

12. Verwendung der thermoplastischen Mischung gemäß den Ansprüchen 1 bis 5 zur Herstellung von Formkörpern zur kontrollierten Freigabe von Wirkstoffen.

13. Verwendung der thermoplastischen Mischung gemäß den Ansprüchen 1 bis 5 zur Herstellung von massiven Formkörpern, Hohlkörpern oder Kombinationen davon.

14. Verwendung der thermoplastischen Mischung gemäß den Ansprüchen 1 bis 5 zur Herstellung von Folien zum Gebrauch in der Landwirtschaft.

15. Verwendung der thermoplastischen Mischung gemäß den Ansprüchen 1 bis 5 zur Herstellung von Folien zum Gebrauch in der Lebensmittelanwendung.

16. Verwendung der thermoplastischen Mischung gemäß den Ansprüchen 1 bis 5 zur Herstellung von Folien zum Gebrauch als Lebensmittelumverpackung.

17. Verwendung der thermoplastischen Mischung gemäß den Ansprüchen 1 bis 5 zur Herstellung von Folien zum Gebrauch als Lebensmittelverpackung mit vollständigem Flächenkontakt zum Lebensmittel.

18. Verwendung der thermoplastischen Mischung gemäß den Ansprüchen 1 bis 5 zur Herstellung von Flach- und tubularen Folien zur Verwendung als Lebensmittelhüllen für Wurst und Käse.

19. Verwendung der thermoplastischen Mischung gemäß den Ansprüchen 1 bis 5 als temporäre Schutzfolie für technische Gegenstände.

## Claims

1. Thermoplastic starch-based mixture obtainable by preparing and mixing
(A) 100 parts by weight of at least one cationic starch a) and at least one anionic starch b), these starches exhibiting a degree of substitution DS of 0.1 - 3.0.
(B) 0 - 400 parts by weight of a thermoplastically processable polymeric material other than A),
(C) water in a quantity which is sufficient for softening the mixture
(D) at least one softener in a quantity of 10 parts by weight up to half the sum of the parts by weight A) and B), and
(E) if necessary, up to ((A) + (B)) parts by weight of further conventional additives,
in which the water content of the components A) and B) has been corrected arithmetically to zero.

2. Thermoplastic mixture according to claim 1, in which the anionic groups have been selected from carboxyl, phosphate, sulphate, borate, phosphonium and sulphonate groups.

3. Thermoplastic mixture according to claim 1 or 2, in which the cationic groups have been selected from tertiary amino, quaternary ammonium, tertiary phosphin, quaternary phosphonium, imino and sulphonium groups.

4. Thermoplastic mixture according to one of claims 1 to 3, in which the ratio of cationic groups to anionic groups is 0.2 - 5.

5. Thermoplastic mixture according to one of claims 1 to 4, in which the starch contains nonionic substituents in addition to the anionic and cationic groups.

6. Method for manufacturing a starch-based thermoplastic mixture, in which
(A) 100 parts by weight of at least one cationic starch a) and at least one anionic starch b), these starches exhibiting a degree of substitution DS of 0.1 - 3.0.
(B) up to 0 - 400 parts by weight of a thermoplastically processable polymeric material other than A),
(C) water in a quantity which is sufficient for softening the mixture
(D) at least one softener in a quantity of 10 parts by weight up to half the sum of the parts by weight A) and B), and
(E) if necessary, up to ((A) + (B)) parts by weight of further conventional additives, in which the water content of the components A) and B) has been corrected arithmetically to zero, are prepared and mixed with each other and the softening takes place with the introduction of thermal and mechanical energy, preferably with increased temperature and simultaneous exertion of shearing forces.

7. Method according to claim 6, in which the softening takes place at temperatures ranging from > 60°C to 200°C.

8. Method according to claim 6 o 7, in which the mixing and/or softening take(s) place under the influence of highly shearing mixing aggregates containing softening elements, and the softening elements exhibit torques ranging from 10 to 100 Nm.

9. Granulate obtainable from the thermoplastic mixture according to claims 1 to 5 by extrusion and pelletisation.

10. Biodegradable moulding obtainable from the plastic mixture according to claims 1 to 5.

11. Use of the thermoplastic mixture according to claims 1 to 5 for manufacturing mouldings or films.

12. Use of the thermoplastic mixture according to claims 1 to 5 for manufacturing moulded bodies for controlled release of active ingredients.

13. Use of the thermoplastic mixture according to claims 1 to 5 for manufacturing solid moulded bodies, hollow bodies or combinations thereof.

14. Use of the thermoplastic mixture according to claims 1 to 5 for manufacturing films for use in agriculture.

15. Use of the thermoplastic mixture according to claims 1 to 5 for manufacturing films for use in the food industry.

16. Use of the thermoplastic mixture according to claims 1 to 5 for manufacturing films for use as food packaging.

17. Use of the thermoplastic mixture according to claims 1 to 5 for manufacturing films for use as food packaging with full surface contact with the food.

18. Use of the thermoplastic mixture according to claims 1 to 5 for manufacturing flat and tubular films for use as food cases for sausage and cheese.

19. Use of the thermoplastic mixture according to claims 1 to 5 as a temporary protective film for technical objects.

## Revendications

1. Mélange thermoplastique à base d'amidon que l'on peut obtenir par préparation et mélange de
(A) 100 parties en poids d'au moins un amidon cationique a) et d'au moins un amidon anionique b), ces amidons présentant un degré de substitution DS de 0,1 à 3,0,
(B) de 0 à 400 parties en poids d'une matière polymère que l'on peut transformer en matière thermoplastique, différente de (A),
(C) eau dans une quantité suffisante pour la plastification,
(D) au moins un plastifiant dans une quantité de 10 parties en poids jusqu'à la moitié de la somme des parties en poids de (A) et (B), et
(E) éventuellement jusqu'à ((A) + (B)) parties en poids d'autres additifs usuels,
où la teneur en eau des composants (A) et (B) est corrigée à zéro par des calculs.

2. Mélange thermoplastique selon la revendication 1, où les groupes anioniques sont pris parmi les groupes carboxyle, phosphate, sulfate, borate, phosphonium et sulfonate.

3. Mélange thermoplastique selon la revendication 1 ou 2, où les groupes cationiques sont pris parmi les groupes amino tertiaire, ammonium quaternaire, phosphine tertiaire, phosphonium quaternaire, imino et sulfonium.

4. Mélange thermoplastique selon l'une des revendications 1 à 3, où le rapport des groupes cationiques aux groupes anioniques est de 0,2 à 5.

5. Mélange thermoplastique selon l'une des revendications 1 à 4, où l'amidon présente en plus des substituants non ioniques des groupes anioniques ou cationiques.

6. Procédé pour la préparation d'un mélange thermoplastique à base d'amidon, dans lequel on prépare et on mélange
(A) 100 parties en poids d'au un amidon cationique a) et d'au moins un amidon anionique b), ces amidons présentant un degré de substitution DS de 0,1 à 3,0,
(B) jusqu'à 400 parties en poids d'une matière polymère que l'on peut transformer en matière thermoplastique, différente de (A),
(C) eau dans une quantité suffisante pour la plastification du mélange,
(D) au moins un plastifiant dans une quantité de 10 parties en poids jusqu'à la moitié de la somme des parties en poids de (A) et (B), et
(E) éventuellement jusqu'à ((A) + (B)) parties en poids d'autres additifs usuels, la teneur en eau des composants (A) et (B) étant corrigée à zéro par des calculs,
et on réalise la plastification par apport d'énergie thermique et mécanique, de préférence à température élevée et par application simultanée des forces de cisaillement.

7. Procédé selon la revendication 6, dans lequel la plastification est mise en oeuvre à une température dans le domaine de >60°C à 200°C.

8. Procédé selon la revendication 6 ou 7, dans lequel le mélange est réalisé sous l'action de mélangeurs présentant des éléments de plastification qui exercent de fortes forces de cisaillement, et les éléments de plastification présentent des moments de couple dans le domaine de 10 à 100 Nm.

9. Granulé que l'on peut obtenir à partir du mélange thermoplastique selon les revendications 1 à 5 par extrusion et pastillage.

10. Profilé ou feuille biodégradable que l'on peut obtenir à partir du mélange thermoplastique selon les revendications 1 à 5.

11. Utilisation du mélange thermoplastique selon les revendications 1 à 5 pour la fabrication de profilés ou de feuilles.

12. Utilisation du mélange thermoplastique selon les revendications 1 à 5 pour la fabrication de corps moulés pour la libération contrôlée de principes actifs.

13. Utilisation du mélange thermoplastique selon les revendications 1 à 5 pour la fabrication de corps moulés, de corps creux ou de leurs combinaisons massives.

14. Utilisation du mélange thermoplastique selon les revendications 1 à 5 pour la fabrication de feuilles pour l'utilisation en agriculture.

15. Utilisation du mélange thermoplastique selon les revendications 1 à 5 pour la fabrication de feuilles pour des applications dans les industries alimentaires.

16. Utilisation du mélange thermoplastique selon les revendications 1 à 5 pour la fabrication de feuilles pour des utilisations comme emballages d'aliments.

17. Utilisation du mélange thermoplastique selon les revendications 1 à 5 pour la fabrication de feuilles utilisées comme emballage d'aliments dont la surface est en contact permanent avec l'aliment.

18. Utilisation du mélange thermoplastique selon les revendications 1 à 5 pour la fabrication de feuilles planes et tubulaires utilisées comme boyaux de saucisson et emballages de fromage.

19. Utilisation du mélange thermoplastique selon les revendications 1 à 5 comme feuille protectrice temporaire d'objets techniques.
